# EUROPEAN PATENT SPECIFICATION

(11) **EP 1 094 830 B1**
(45) Date of publication and mention of the grant of the patent: **21.09.2005**
(21) Application number: 99933439.4
(22) Date of filing: 05.07.1999
(51) Int. Cl.: A61K 38/09, A61P 25/00

(54) **USE OF COMPOSITION COMPRISING AT LEAST ONE SUBSTANCE WITHIN THE GROUP GnRH-ANALOGUES FOR TREATMENT OF OBSESSIVE-COMPULSIVE DISORDER (OCD)**
VERWENDUNG EINER ZUSAMMENSETZUNG ENTHALTEND ZUMINDEST EIN GNRH-ANALOGES ZUR BEHANDLUNG VON ZWANGSNEUROTISCHEN ERKRANKUNGEN
UTILISATION D'UNE COMPOSITION RENFERMANT AU MOINS UNE SUBSTANCE SELECTIONNEE DANS LE GROUPE DES ANALOGUES DE LA GnRH POUR LE TRAITEMENT DES TROUBLES OBSESSIONELS IMPULSIFS

(30) Priority: 07.07.1998 SE 9802438
(43) Date of publication of application: 02.05.2001
(73) Proprietor: Läkartjänster I Västsverige AB, 427 22 Billdal (SE)
(72) Inventor: ERIKSSON, Tomas, S-427 22 Billdal (SE)
(74) Representative: Cederbom, Hans Erik August
(86) International application number: PCT/SE1999/001218
(87) International publication number: WO 2000/001403

(56) References cited:
- WO-A-00/12115
- ACTA PSYCHIATR. SCAND. CASA M. ET AL: 'Antiandrogenic Treatment of Obsessive-Compulsive Neurons' vol. 73, 1986, SPAIN, pages 221 - 222, XP000863589
- Thibaut et al: 'Gonadotrophin hormone releasing hormone agonist in cases of severe paraphilia: a lifetime treatment?', Psychoneuroendocrinology, Vol. 21, Issue 4, May 1996, pages 411-419

## Description

### TECHNICAL FIELD

The present invention relates to the use of at least one substance within the group of GnRH-analogues for the treatment of obsessive-compulsive disorder (OCD).

### STATE OF THE ART

OCD is a chronic psychiatric disease in which the main symptoms are recurrent obsessions or compulsions that are severe enough to be time-consuming or cause marked distress or significant impairment. They are often painful and destructive and prevent the person from thinking of other things or devoting himself/herself to other activities. The disorder is usually chronic and often serious enough to make the patient completely or partially unable to work and live a normal life.

The disorder is described and defined in detail in The Diagnostic and Statistical Manual of Mental Disorders, fourth edition (DSM-IV) published by the American Psychiatric Association in 1994.

At the present state of the art of science, the only pharmacological treatments which are considered to be effective for the treatment of OCD, are such pharmacological agents which increase the activity in those neurones in the central nervous system in which serotonin (5-HT; 5-hydroxytryptamine) serve as neurotransmitter. Principally, there are two groups of such pharmacological agents:
1) Certain tricyclic antidepressive agents, especially chlomipramine (Anafranil®);
2) Selective serotonin reuptake inhibitors (SSRI), e.g. fluvoxamin (Fevarin®).

That this is the present scientific opinion is evident from the following Swedish and English psychiatric textbooks.
1. Ottosson, J-O: Psykiatri. Almqvist & Wiksell Medicin Liber Utbildning. Stockholm 1995. Chapter 15. Pages 371-384.
2. Gelder, M., Gath, D., Mayou, R., Cowen, P.: Oxford Textbook of Psychiatry. Oxford Medical Publications. Oxford 1996. Pages 180-186.

Another method used to treat OCD-patients, who have not responded to pharmacological treatment, is a surgical operation in the brain, which sometimes can provide relief of OCD-symptoms. This method involves the leasioning of certain nerve paths in the brain. Normally, a few patients are operated in this way each year in Sweden. The method has a very limited use because of the risk of serious side effects. One common side effect is increase in weight, also there are cases of apathy and reduced memory function.

### Physiological regulation of androgenic hormones under normal conditions (i.e. without influence of drugs)

Gonadotropin-releasing hormone (GnRH) stimulates the production of gonadotropins in the hypophysis (at the bottom of the brain). The gonadotropins are released to the blood and transported to the testes and the adrenal glands (of the male) and to the ovaries and the adrenal glands (of the female). There, the gonadotropins stimulate the synthesis and release of, among other hormones, the androgens (the male sex hormones) of which testosterone is one.

The androgenic hormones are released to the blood from the glands in which they are produced. They are transported to different organs where they exert their various actions. One of these organs is the brain. There, the androgenic hormones exert their effects by stimulating certain receptors. The androgenic activity in the brain is dependent both on the concentration of androgenic hormones in the blood, and on the density and sensitivity of the receptors on which the androgenic hormones act. The androgenic activity may thus be high, both at a high concentration of androgenic hormone in the blood, and in cases of a high density and/or sensitivity of the androgenic receptors.

The production of androgenic hormones is normally subjected to a "feed-back" regulation. If the androgenic activity in the brain is high, a compensating reduction in the synthesis and release of GnRH and consequently of the of gonadotropins takes place, with a following reduction in the synthesis and release of androgenic hormones. At a high androgenic activity in the brain, caused by a high density and/or sensitivity of the receptors (and not due to a high concentration of androgenic hormones in the blood), the compensating feed-back-regulation might cause a decrease in the synthesis and release of androgenic hormones which in turn might cause abnormally low blood concentrations of androgenic hormones. Such low concentrations could not be taken as a sign of low androgenic activity; it may still be high (if the compensation has not been sufficient) or normal (if the compensation has been sufficient).

### Known fields of application for pharmacological agents which reduce the activity of androgenic hormones

There are several drugs which have well-documented anti-androgenic effects. These drugs are mainly used in the treatment of carcinoma of the prostate but also in the treatment of unwanted phenomena, which are caused by a high androgenic activity. Examples of such phenomena are hypersexuality and male pattern baldness in men and hirsutism (a condition with bodily male hair growth) in women.

Furthermore, in the scientific literature there is a paper in which it has been suggested that anti-androgenic treatment might be effective in the treatment of OCD. This work is: Casas, M. et al: Antiandrogenic treatment of obsessive-compulsive neurosis. Acta psychiatr. Scand. 1986. Vol 73. Pages 221-222.

That paper reports randomly made observations on patients treated with cyproterone acetate. This compound has a different mode of action than GnRH-analogues. It is, however, on theoretical grounds likely that cyproterone acetate may have a certain effect upon OCD, since also this drug has anti-androgenic properties. However, the observation by Casas and colleagues has not been reproduced by any group which has published any such observations. Instead, there is in the scientific literature a publication by a group which has not been able to reproduce the observation that cyproterone acetate might be effective in the treatment of OCD.

### Groups of drugs with anti-androgenic properties

Anti-androgenic effects may be obtained via three different mechanisms:

### Receptor-blocking substances

These are substances that bind to the androgenic receptors without stimulating them. In this way, the endogenous hormone is prevented from binding to the receptors with a resulting reduced androgenic activity. Preparation example: flutamide (Eulexin®).

### Synthesis-inhibiting substances

These are substances which inhibit production of androgenic hormones in the glands where they are produced. Preparation example: cyproterone acetate (Androcur®) (this substance also has receptor-blocking properties).

### GnRH-analogues

These are substances which, in their effect, resemble endogenous GnRH but they produce a more powerful and more protracted effect upon the GnRH-receptors. Initially they have the same effects as endogenous GnRH, i.e. by stimulating the production of gonadotropins, the synthesis and release of androgenic hormones is also stimulated resulting in an increase in androgenic activity. After treatment during a certain period of time the sensitivity in the receptors, on which endogenous GnRH and GnRH-analogues act, will be significantly reduced. Hereby, the release of gonadotropins will also be reduced. The reduction in concentrations of circulating gonadotropins will cause a decrease in synthesis and release of androgenic hormones. Consequently, the androgenic activity in the brain and elsewhere will be dramatically reduced.

Preparation example: buserelin (Suprecur®; manufacturer Hoechst Marion Roussel), leuprorelin (Enanton®; manufacturer Orion and Procren®; manufacturer Abbott), goserelin (Zoladex®; manufacturer Zeneca), triptorelin (Decapeptyl®; manufacturer Ferring).

### SUMMARY OF THE INVENTION

One object of the present invention is to provide a pharmaceutical medicament, which enables for patients with OCD to obtain partial or complete relief of symptoms by treatment with this composition.

### THE SOLUTION

For this object, the invention is characterised in that the medicament comprises at least one substance within the group GnRH-analogues.

Different variants of the invention are disclosed in the accompanying depending claims.

### PRIOR ART

By the document; Antiandrogenic Treatment of Obsessive-Compulsive Neurons', 'CASA M.ET AL', 'ACTA Psychiatr.Scand.', 'SPAIN', 73//00-00-1986, 221-222, it has been shown that one pharmacological agent (cyproterone acetate) with anti-androgenic properties has been effective in the treatment of a few patients with obsessive-compulsive neurosis (nowadays called obsessive-compulsive disorder, OCD).

This observation has never been confirmed. On the contrary, in later investigations the researchers have failed to demonstrate any effect of cyproterone acetate on OCD.

In our investigation we have used a pharmacological agent (a long acting analogue of the gonadotropins releasing hormone= GnRH analogue with a completely different mode of action. While cuproterone acetate influences the synthesis and release of androgenic hormones from the hormone-producing glands, GnRH analogues exert their effects in the brain where they prevent the release of luteinizing hormone, LH, which stimulates the synthesis of androgens in the periphery.

GnRH-analogues differ from cyproterone acetate not only in the mode of action. They also cause a total blockage of the androgen system while cyproterone acetate only is capable of causing a partial inhibition of this system.

Furthermore, in our invention, the GnRH-analogue must be used for several months before any effect could be demonstrated. This is an absolutely new approach in the use of pharmacological agents with anti-androgenic properties.

Moreover, the patients, who have been successfully treated with GnRH-analogues by us were previously treated with cyproterone acetate, also by us, without success.

Thus, our invention differs from what is reported in D1 in the following respects:
1. GnRH-analogues exert their anti-androgenic effects by a quite different mode of action than does cyproterone acetate.
2. Our invention includes the use of GnRH-analogues for several months. Such a long treatment period has never before even been discussed.
3. GnRH-analogues, but not cyproterone acetate, is effective in severe cases of OCD.

By the document: Thibaut et al.: 'gonadotrophin hormone releasing hormone agonist in cases of severe paraphilia: a lifetime treatment?', Psychoneuroendocrinology, Vol. 21, Issue 4, May 1996, pages 411-419 It is demonstrated that GnRH-analogues are effective in the treatment of patients with various forms of paraphilia.

Paraphilia are various forms of deviant sexual behaviour, such as e.g. pedophilia.

Since GnRH-analogues, and also any other pharmacological agents which inhibits the release of androgenic sexual hormones, inhibits any sexual behaviour it is not surprising in any way that these compounds also inhibits various forms of paraphilia.

OCD, and various forms of paraphilias, have, however, never been considered to be the same psychiatric disorder. On the contrary, in the systematisation of psychiatric disorders they represent different groups of disorders.

The present invention, described in the following, has its intellectual basis in observations that have been made in contacts with patients at a psychiatric specialist clinic, in combination with established scientific facts. To sum up, the following observations and facts are applied.
1. Remarkably many patients with OCD have been found to have clinical symptoms of a high androgenic activity such as a strong sexual drive, a sebaceous skin and a male pattern baldness.
2. On the basis of observation (1) has, at the above mentioned clinic, as a clinical routine examination, the determination of the free fraction of the androgenic hormone testosterone in blood been introduced. Hereby, it has been noticed that patients suffering from serious forms of OCD have subnormal blood concentrations of free testosterone. This finding has been interpreted as an effect secondary to an increased androgenic activity in the brain.
3. A patient who has been suffering from a very severe form of OCD which has made him totally disabled and for which he has been receiving sickness pension for over ten years, has after three months of treatment with the GnRH-analogue triptorelin become substantially recovered. The patient had previously been treated with cyproterone acetate without any major improvement.

The treatment of OCD in accordance with the invention is performed in the following manner:

The following conditions must be fulfilled in order for the described treatment to be appropriate.
1. The diagnosis should be ensured in accordance with the criteria specified for example in DSM-IV.
2. Those established treatments, which do not cause severe side effects, should first have been tried or at least considered.
3. The patient should have been judged to be so severely plagued or otherwise handicapped by the disease that it is obvious that the advantage of a possible cure or relief of symptoms balances the disadvantage of the side effects of the treatment. The side effects here referred to are the reduction of the sexual drive and the reduced sexual performance, which may be expected as a result of the treatment.
4. Before the pharmacological treatment starts, the patient must be subjected to a medical investigation to make certain that he/she is not suffering from any other serious disease.

In the treatment, the patient is repeatedly given a pharmacological agent from the pharmaceutical preparation group GnRH-analogues which, by decreasing the sensitivity in the receptors controlling the synthesis and release to the blood of the gonadotropins, cause a marked decrease in synthesis and release of androgenic hormones to the blood. Hereby the androgenic activity is dramatically reduced. There are several substances available on the market which cause the desired effects. There are also alternative pharmaceutical preparations available on the market. Thus, there are presently preparations for various forms of injection and for administration by nasal spray. From a general point of view it is probable that, in the treatment of OCD, these pharmacological agents should be administered in the same way and in the same doses as when they are used in the treatment of carcinoma of the prostate.

One example of an administration procedure for one of these pharmacological agents is delivered in the following.

The GnRH-analogue triptorelin is available in a pharmaceutical preparation (Decapeptyl® Depot). In the use of this preparation, an injection is administered containing triptorelin embonate in a dose corresponding to 3.75 mg triptorelin. Injections with this dose are given intramuscularly. They should be repeated every fourth week from the first injection.

In order to verify that a sufficient effect has been achieved, the blood concentration of free testosterone should be assessed, e.g. once every fortnight until full effect has been established. This is the case when the blood concentration of free testosterone has fallen to levels below the detection limit or very close to it.

Initially during the treatment there is an increase in the amount of circulating androgenic hormones in the blood and consequently in androgenic activity. This increase, which may remain for some weeks, may result in a change for the worse in the OCD-symptoms. This impairment could be expected to remain until the androgenic activity has been reduced. This could be expected to take some weeks. To avoid the risk of such an initial deterioration, the patient may, during the first phase of the treatment, be treated also with the androgenic receptor-blocking agent flutamide (Eulexin®) in a dose of 250 mg administrated three times each day in tablets. These tablets are commercially available for use against carcinoma of the prostate. However, this additional treatment, is not necessary for the efficiency of the above described treatment of OCD; it has the sole purpose of avoiding the risks of an initial accentuation of the symptoms.

The here described pharmacological treatment of OCD implies, as in all other psychiatric treatments, that the psychiatrist provides the patient with a continuous psychological support.

Presently, it is not possible to say whether the treatment must be chronically administered, or if it could be terminated after a certain period of time without risking relapses into OCD symptoms. This question must be answered in coming clinical investigations.

### EXAMPLE

A detailed case study of the treatment of a patient with a severe form of OCD is disclosed in the following, as supporting evidence of the effect of the method.

A then 43 year old man was referred to a psychiatric specialist clinic from another qualified psychiatrist for evaluation and treatment of a severe condition of OCD. The reason for referring the patient to the clinic was that the referring psychiatrist, after several years of treatment, without any improvement in the OCD-symptoms of the patient, did not find that there was anything more he could do for the patient.

The situation when the patient came to the clinic was that he had sickness pension since more than ten years. He lived alone in a small apartment which he hardly ever left. Obsessions and compulsive rituals occupied most of his time. He repeated against his will meaningless strings of words silently during several hours every day. He felt he had to read the newspaper according to certain ritual patterns; if he did anything wrong, he had to start again from the beginning. Sometimes, it took him the whole day to read the newspaper. He also arranged his possessions according to certain meaningless rules. Among other things, he placed his books so that the covers were strictly oriented along north-south lines. His contacts with other persons were highly restraint and mainly limited to some relatives.

At the initial examination of the patient, he was found to be severely ill and extremely tormented by the disease. The clinical diagnosis was that he suffered from a very serious form of OCD. He was also found to be intelligent without any signs of any other mental illness or personality disorder. Thus, it was a clearly defined case of OCD.

Certain symptoms of androgenic hyperactivity such as a male pattern baldness, a very sebaceous skin with a strong disposition towards developing acne and a sexual drive in the upper region of the normal range were also found at the initial examination. All these symptoms were also problematic for him.

An endocrinological investigation was performed, based upon his symptoms of androgenic hyperactivity. This disclosed a low blood concentration of free testosterone; 38 pmol/litre (normal range for his age is reported to be 55-114 pmol/litre). Since the patient had symptoms of androgenic hyperactivity this laboratory finding could, however, not be a symptom of a low androgenic activity. Instead, the interpretation of this laboratory finding was that it was a sign of a compensatory reduction in the synthesis and release of testosterone occurring as a consequence of an increased androgenic activity in the brain.

During long periods of time the patient had been treated with the drugs which presently are considered to have the best effect against OCD. In the treatment of the patient he had also been given supportive psychotherapy.

Treatment against the patients symptoms of androgenic hyperactivity has also been given with the anti-androgenic drugs cyproterone acetate (Androcur®) and flutamide (Eulexin®), without any significant effects on his symptoms of androgenic hyperactivity and without any obvious effect on his OCD.

Thus, it was assumed that the patient had a very strong androgenic hyperactivity. In order to overcome this, treatment with the long-acting GnRH-analogue triptorelin (Decapeptyl® Depot) was given every fourth week as deep intramuscular depot injections with a dose corresponding to 3.75 mg triptorelin in each injection. Initially, the treatment caused an accentuation of the patients OCD-symptoms. That result could hardly be interpreted in any other way than that the treatment, during the period when it could be expected to increase the androgenic activity also increased the OCD-symptoms. After two months treatment, the patient began to feel better. After another two months, he was dramatically better; all obsessive thoughts had disappeared and the patient became subject for rehabilitation with the aim of making it possible for him to work or study.

It is absolutely clear that and the dramatic improvement of the patient was caused by the treatment with a GnRH-analogue.

The invention is not limited to the above described embodiments, but several variants are conceivable within the scope of the accompanying claims.

## Claims

1. Use of at least one substance within the group of GnRH-analogues for the manufacture of a medicament for treatment of obsessive-compulsive disorder (OCD).

2. Use according to claim 1, wherein the medicament is apart of a preparation which is intended for administration by means of subcutaneous injection.

3. Use according to claim 1, wherein the medicament is a part of a preparation which is intended for administration by means of a nasal spray.

4. Use according to claim 1, wherein the medicament is part of a preparation which is intended for administration by means of intramuscular injection.

5. Use according to any one of claim 1-4, wherein the GnRH-analogue is combined with a substance which blocks the androgenic receptors in the brain.

6. Use according to claim 5, wherein the blocking substance consists of flutamide.

7. Use according to any one of claim 1-6, wherein the GnRH-analogue consists of triptorelin.

8. Use according to claim 7, wherein a dose of injection contains triptorelin embonate in a quantity corresponding to 3.75 mg triptorelin.

9. Use according to claim 7, wherein the dose of injection is intended to be repeated with an interval of about four weeks.

10. Use according to any one of claims 1-6, wherein the GnRH-analogue consists of buserelin, leuprorelin or goserelin.

## Patentansprüche

1. Verwendung wenigstens einer Substanz innerhalb der Gruppe von GnRH-Analogen für die Herstellung eines Medikamentes zur Behandlung von zwangsneurotischen Erkrankungen.

2. Verwendung entsprechend Anspruch 1, bei welcher das Medikament ein Teil einer Präparation ist, die zur Applikation mittels subkutaner Injektion vorgesehen ist.

3. Verwendung entsprechend Anspruch 1, bei welcher das Medikament ein Teil einer Präparation ist, die zur Applikation mittels eines Nasensprays vorgesehen ist.

4. Verwendung entsprechend Anspruch 1, bei welcher das Medikament ein Teil einer Präparation ist, die zur Applikation mittels intramuskulärer Injektion vorgesehen ist.

5. Verwendung entsprechend irgendeinem der Ansprüche 1 - 4, bei welcher das GnRH-Analoge mit einer Substanz kombiniert ist, welche androgene Rezeptoren im Gehirn blockiert.

6. Verwendung entsprechend Anspruch 5, bei welcher die blockierende Substanz aus Flutamid besteht.

7. Verwendung entsprechend irgendeinem der Ansprüche 1 - 6, bei welcher das GnRH aus Triptorelin besteht.

8. Verwendung entsprechend Anspruch 7, bei welcher eine Injektionsdosis Triptorelinambonat in einer Menge enthält, die 3,75 mg Triptorelin entspricht.

9. Verwendung entsprechend Anspruch 7, bei welcher die Injektionsdosis zur Wiederholung mit einem Intervall von etwa 4 Wochen vorgesehen ist.

10. Verwendung entsprechend irgendeinem der Ansprüche 1 - 6, bei welcher das GnRH-Analoge aus Buserelin, Leuprorelin oder Goserelin besteht.

## Revendications

1. Utilisation d'au moins une substance parmi le groupe des analogues de la gonadolibérine (LH-RH) pour produire un médicament destiné au traitement des troubles obsessionnels compulsifs (TOC).

2. Utilisation selon la revendication 1, dans laquelle le médicament fait partie d'une préparation destinée à être administrée en injection sous-cutanée.

3. Utilisation selon la revendication 1, dans laquelle le médicament fait partie d'une préparation destinée à être administrée au moyen d'un pulvérisateur nasal.

4. Utilisation selon la revendication 1, dans laquelle le médicament fait partie d'une préparation destinée à être administrée en injection intramusculaire.

5. Utilisation selon l'une quelconque des revendications 1 à 4, dans laquelle l'analogue de la LH-RH est combiné avec une substance qui bloque les récepteurs androgènes dans le cerveau.

6. Utilisation selon la revendication 5, dans laquelle la substance de blocage se compose de flutamide.

7. Utilisation selon l'une quelconque des revendications 1 à 6, dans laquelle l'analogue de la LH-RH se compose de triptoréline.

8. Utilisation selon la revendication 7, dans laquelle une dose d'injection contient de l'embonate de triptoréline selon une quantité correspondant à 3,75 mg de triptoréline.

9. Utilisation selon la revendication 7, dans laquelle la dose d'injection est destinée à être répétée à intervalle d'environ quatre semaines.

10. Utilisation selon l'une quelconque de revendications 1 à 6, dans laquelle l'analogue de la LH-RH se compose de buséréline, de leuproréline ou de goséréline.
